(19) **Europäisches Patentamt / European Patent Office / Office européen des brevets**

(11) **EP 2 662 855 A1**

(12) **EUROPEAN PATENT APPLICATION**
published in accordance with Art. 153(4) EPC

(43) Date of publication:
**13.11.2013 Bulletin 2013/46**

(21) Application number: **11855034.2**

(22) Date of filing: **04.01.2011**

(51) Int Cl.:
**G10L 21/02** $^{(2013.01)}$

(86) International application number:
**PCT/JP2011/050017**

(87) International publication number:
**WO 2012/093470 (12.07.2012 Gazette 2012/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(71) Applicant: **Fujitsu Limited**
**Kawasaki-shi, Kanagawa 211-8588 (JP)**

(72) Inventors:
• **SUZUKI, Masanao**
  **Kawasaki-shi, Kanagawa 211-8588 (JP)**

• **OTANI, Takeshi**
  **Kawasaki-shi, Kanagawa 211-8588 (JP)**
• **TOGAWA, Taro**
  **Kawasaki-shi, Kanagawa 211-8588 (JP)**
• **ISHIKAWA, Chisato**
  **Kawasaki-shi, Kanagawa 211-8588 (JP)**

(74) Representative: **HOFFMANN EITLE**
**Patent- und Rechtsanwälte**
**Arabellastrasse 4**
**81925 München (DE)**

(54) **VOICE CONTROL DEVICE, VOICE CONTROL METHOD AND VOICE CONTROL PROGRAM**

(57) A voice control device includes a hearing estimate section configured to estimate hearing of a user based on a sending/received sound ratio representing a ratio of the volume of a sending sound to the volume of a received sound; a compensation-quantity calculating section configured to calculate a compensation quantity for a received signal of the received sound responsive to the estimated hearing; and a compensation section configured to compensate the received signal based on the calculated compensation quantity.

FIG.2

**Description**

[Technical Field]

The invention relates to a voice control device, a voice control method, a voice control program and a portable terminal device that control received sound.

[Background Art]

Conventionally, there are portable terminal devices that execute control to make received voices easy to hear. For example, there is a technology with which multiple single-tone frequency signals are reproduced for a user to calculate the minimum hearing level based on the user's hearing result for processing a voice (Patent document 1).
Also, there is a technology in which a Lombard effect is utilized so that if a sending sound volume is loud, it is determined that it is noisy in the surroundings to increase the received sound volume, if a sending sound volume is soft, the received sound volume is decreased automatically (Patent document 2).
Also, there is a technology in which an equalizer is provided for emphasizing a voice signal in a specific range, and characteristics of the equalizer are adjusted based on a volume operation by a user (Patent document 3).

[Related-Art Documents]

[Patent document]

[Patent document 1] Japanese Laid-open Patent Publication No. 07-66767
[Patent document 2] Japanese Laid-open Patent Publication No. 2004-165865
[Patent document 3] Japanese Laid-open Patent Publication No. 2010-81523

However, there is a problem with Patent document 1 in that it is not easy to use because a user needs to execute a hearing test that forces a complicated procedure upon the user.
Also, there is a problem with Patent document 2 in that there are cases where sound quality may be bad depending on a user because the received sound volume is determined only by the sending sound volume, hence a hearing characteristic of the user is not taken into account.
Also, there is a problem with Patent document 3 in that voice control may not be carried out during a call because it requires a user to do a volume operation, which is, however, difficult to do during a call.

[Summary of the Invention]

[Problem to be Solved by Invention]

Thereupon, in view of the above problems, it is an object of the technology disclosed herein to provide a voice control device, a voice control method and a voice control program with which voice control can be executed in response to a user's hearing without forcing a burden on the user.

[Means to Solve the Problem]

According to one embodiment, a voice control device includes a hearing estimate section configured to estimate hearing of a user based on a sending/received sound ratio representing a ratio of the volume of a sending sound to the volume of a received sound; a compensation-quantity calculating section configured to calculate a compensation quantity for a received signal of the received sound responsive to the estimated hearing; and a compensation section configured to compensate the received signal based on the calculated compensation quantity.

[Advantage of the Invention]

According to the disclosed technology, it is possible to execute voice control in response to a user's hearing without forcing a burden on the user.

[Brief Description of Drawings]

FIG. 1 is a schematic view illustrating a change of hearing level with aging;
FIG. 2 is a block diagram illustrating an example of functions of a voice control device according to an embodiment;
FIG. 3 is a block diagram illustrating an example of a hearing estimate section;
FIG. 4 is a schematic view illustrating an example of a relationship between sending/received sound ratio and age;
FIG. 5 is a schematic view illustrating an example of a relationship between age and minimum audible range;
FIG. 6 is a block diagram illustrating an example of a configuration of a hearing compensation section;
FIG. 7 is a schematic view illustrating an example of a relationship between noise quantity and compensation quantity;
FIG. 8 is a schematic view illustrating an example of minimum audible ranges before and after compensation;
FIG. 9 is a schematic view illustrating an example of a relationship between noise quantity and minimum audible ranges before and after compensation;
FIG. 10 is a schematic view illustrating spectrum compensation;
FIG. 11 is a flowchart illustrating an example of a voice control procedure according to the embodiment;
FIG. 12 is a flowchart illustrating an example of a hearing compensation procedure; and
FIG. 13 is a block diagram illustrating an example of hardware of a portable terminal device according to an embodiment.

[Description of Reference Symbols]

101, 102    frequency transform section
103         hearing estimate section
104         noise estimate section
105         hearing compensation section
106         spectrum compensation-quantity calculating section
107         spectrum compensation section
108         inverse frequency transform section
204         control section

[Mode for Carrying Out the Invention]

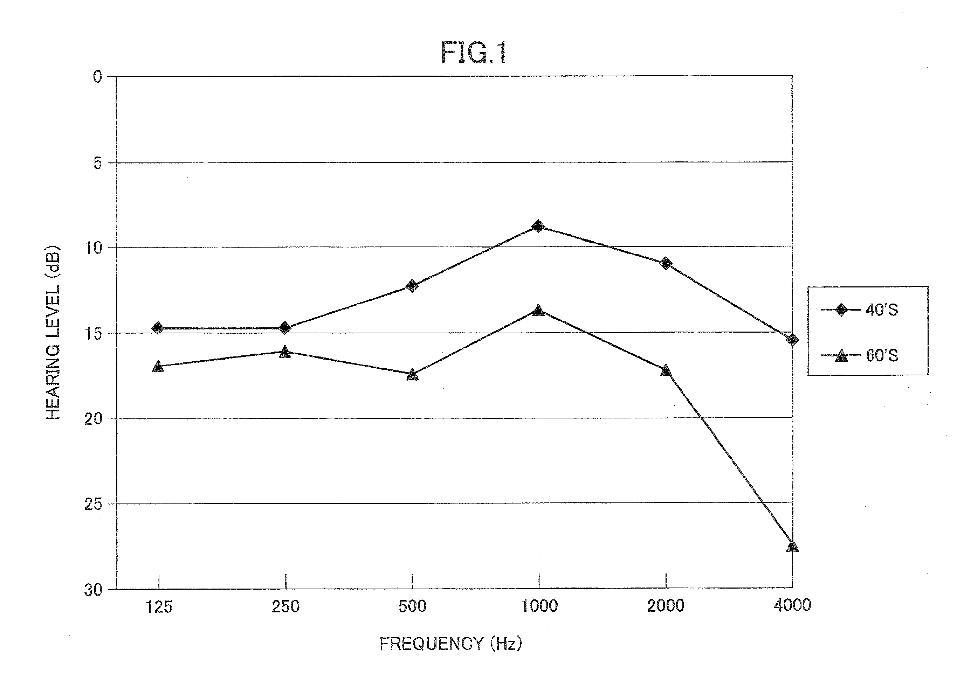

First, a relationship between age and hearing will be described. Hearing is, for example, a minimum audible range. FIG. 1 is a schematic view illustrating a change of hearing level with aging. According to an experiment illustrated in FIG. 1, it can be understood that there is a difference in the average hearing level between age 40 to 60. The hearing level of 60's notably drops in a high range (2 kHz to 4 kHz). Also, as for hearing impairment induced by aging, an investigation result was reported in Http://tri-osaka.jp/group/infoele/life/sense/data/katagiri/0706 22.pdf.

As illustrated in FIG. 1, it is already  known that hearing level drops with aging. Especially, in a high frequency range, hearing level drops when one gets older.

Here, the Lombard effect will be described. the Lombard effect is an effect that if it is noisy in the surroundings, or it is difficult to hear a voice of a counterpart because the counterpart is a quiet talker, one's speaking voice becomes louder. For example, it has been investigated that if background noise is 50 dBSPL (simply denoted as dB hereafter), the speaking volume becomes 4 dB greater than in a quiet situation (37 dB). As for this investigation, refer to FIG. 1 in "Effects of noise on speech production: acoustic and perceptual analyses", W. Van. Summers et. al., J. Acoust. Soc. Am., Vol.84, No.3, September 1988.

However, the Lombard effect affects not only volumes of the surrounding noise and the voice of a counterpart, but also the hearing of a listener. If hearing is reduced, it becomes harder to hear a voice of a counterpart, hence the speaking voice tends to become louder. As illustrated in FIG. 1, hearing reduction is related to age, hence there is a relationship between the Lombard effect and age.

Therefore, in the following, embodiments will be described in which The Lombard effect is used to obtain a relationship between a received sound volume and a sending sound volume, in which age is estimated to estimate hearing for controlling the received sound to make the received voice easier to hear. The embodiments will be described below with reference to the drawings.

[Embodiment]

<Configuration>

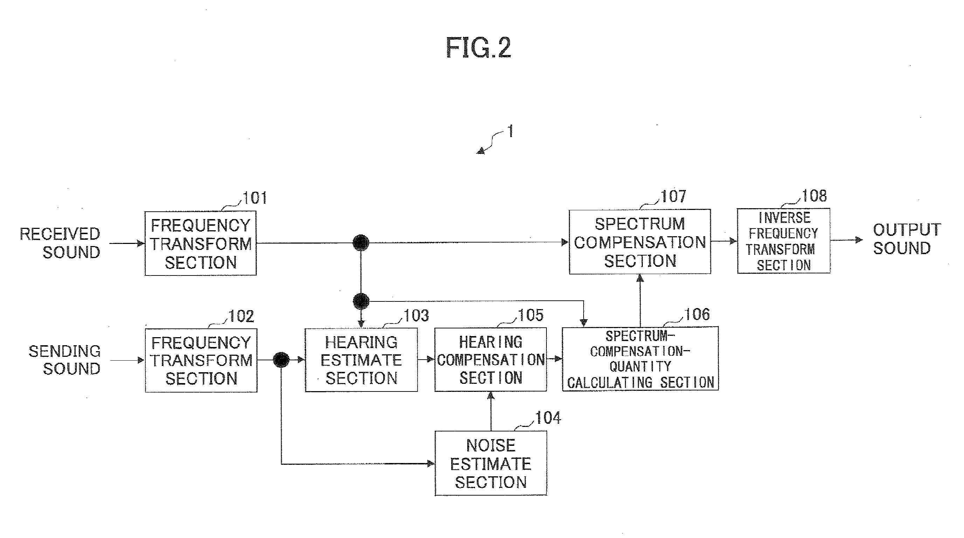

A configuration of a voice control device 1 will be described according to the embodiment. FIG. 2 is a block diagram illustrating an example of a configuration of the voice control device 1 according to the embodiment. As illustrated in FIG. 2, the voice control device 1 includes frequency transform sections 101 and 102, a hearing estimate section 103, a noise estimate section 104, a hearing compensation section 105, a spectrum compensation-quantity calculating section 106, a spectrum compensation section 107, and an inverse frequency transform section 108.

The time-frequency transform section 101 applies a time-frequency transform to a received signal r(t) of a received sound to obtain a spectrum R(f) according to the following formula (1). The time-frequency transform is, for example, a fast Fourier transform (FFT).

$$R(f) = \mathrm{Re}\{R(f)\} + j \cdot \mathrm{Im}\{R(f)\} \qquad \cdots \text{FORMULA 1}$$

f: frequency (f = 0, 1, 2, ..., K-1)
K: Nyquist frequency
Re { } : real part
Im {} : imaginary part

The frequency transform section 101 outputs the obtained spectrum R(f) to the hearing estimate section 103, the spectrum compensation-quantity calculating section 106, and the spectrum compensation section 107.

The time-frequency transform section 102 applies a time-frequency transform to a sending signal s(t) of a sending sound to obtain a spectrum S(f) according to the following formula (2). The time-frequency transform is, for example, a fast Fourier transform (FFT).

$$S(f) = \mathrm{Re}\{S(f)\} + j \cdot \mathrm{Im}\{S(f)\} \qquad \cdots \text{FORMULA 2}$$

f: frequency (f = 0, 1, 2, ..., K-1)
K: Nyquist frequency
Re {} : real part
Im {} : : imaginary part

The frequency transform section 102 outputs the obtained spectrum S(f) to the hearing estimate section 103 and the noise estimate section 104.

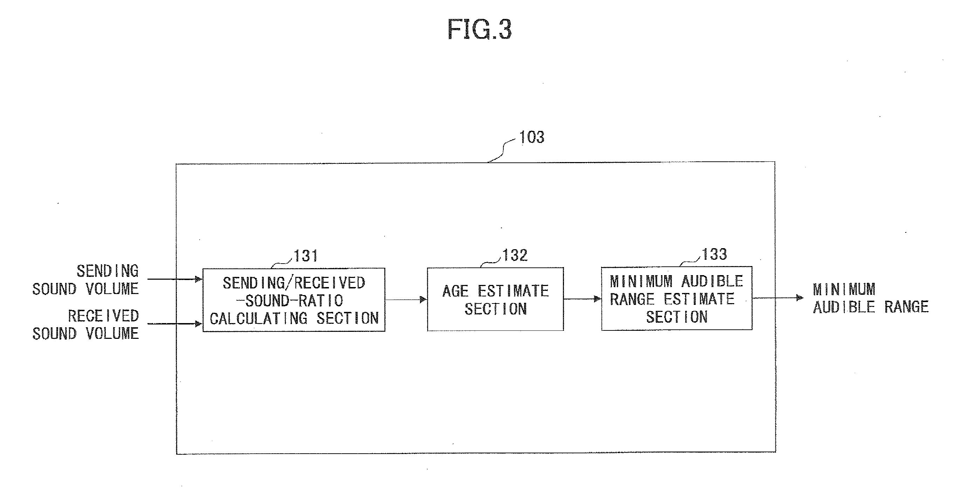

The hearing estimate section 103 estimates a user's hearing based on the received sound volume and the sending sound volume. FIG. 3 is a block diagram illustrating an example of the hearing estimate section 103. In an example illustrated in FIG. 3, the hearing estimate section 103 includes a sending/received-sound-ratio calculating section 131, an age estimate section 132, and a minimum audible range estimate section 133.

The sending/received-sound-ratio calculating section 131 calculates the average electric power of the spectrum R(f) of the received sound and the spectrum S(f) of the sending sound by the following formula.

$$R\_ave = \frac{1}{K}\sum_{f=0}^{K-1}|R(f)|^2 = \frac{1}{K}\sum_{f=0}^{K-1}\left[|\mathrm{Re}\{R(f)\}|^2 + |\mathrm{Im}\{R(f)\}|^2\right] \qquad \cdots \text{FORMULA 3}$$

$$S\_ave = \frac{1}{K}\sum_{f=0}^{K-1}|S(f)|^2 = \frac{1}{K}\sum_{f=0}^{K-1}\left[|\mathrm{Re}\{S(f)\}|^2 + |\mathrm{Im}\{S(f)\}|^2\right] \qquad \cdots \text{FORMULA 4}$$

R_ave: average electric power of the spectrum of the received sound
S_ave: average electric power of the spectrum of the sending sound

The sending/received-sound-ratio calculating section 131 obtains a sending/received sound ratio sp_ratio, for example, from the average power R_ave of the received sound and the average power S_ave of the sending sound by the following formula.

$$sp\_ratio = S\_ave / R\_ave \qquad \cdots \text{ FORMULA (5)}$$

sp_ratio: sending/received sound ratio

The sending/received-sound-ratio calculating section 131 sets the sending/received sound ratio to the ratio of the volume of the received sound to the volume of the sending sound. The sending/received-sound-ratio calculating section 131 outputs the obtained sending/received sound ratio to the age estimate section 132.

Having obtained the sending/received sound ratio from the sending/received-sound-ratio calculating section 131, the age estimate section 132 estimates the age of a user with referring to information that indicates a relationship between the sending/received sound ratio and age, which is stored beforehand.

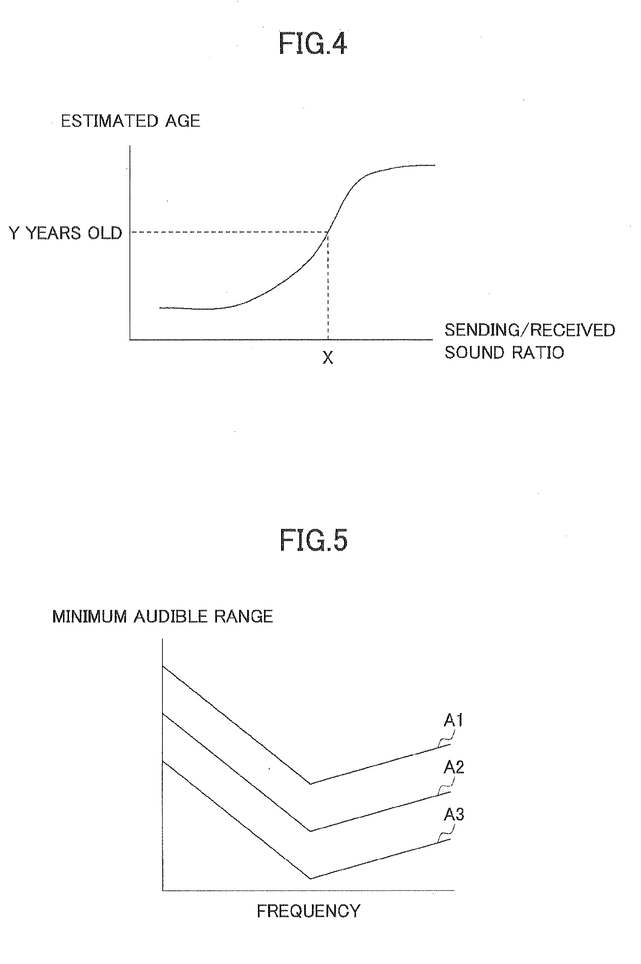

FIG. 4 is a schematic view illustrating an example of a relationship between a sending/received sound ratio and age. An experiment has been executed based on a notion that when one gets older, hearing is impaired, which makes it difficult to hear a voice of a counterpart, hence a speaking voice becomes louder. By the experiment similar to that for obtaining the Lombard effect, a relationship between age and the sending/received sound ratio has been estimated beforehand.

For example, a relationship between age and the sending/received sound ratio can be estimated with the following steps:

(1) For each age (or by generation such as teenagers, twenties), estimate sending sound volume with respect to received sound volume (for example, 60 dB) with an examinee.
(2) Obtain the average sending sound volume of all examinees of the ages measured in (1).
(3) Obtain a ratio of the average sending sound volume in (2) to the received sound volume (sending/received sound ratio).
(4) Execute the steps (1) to (3) for other values of the received sound volume (for example, 30 to 80 dB).

Thus, the information that indicates a relationship between age and the sending/received sound ratio can be obtained for the values of the received sound volume. The age estimate section 132 holds the information that indicates a relationship between age and sending/received sound ratio.

Based on the sending/received sound ratio obtained from the sending/received-sound-ratio calculating section 131, the age estimate section 132 estimates an age from the relationship illustrated in FIG. 4. For example, the age estimate section 132 identified the information indicating the relationship illustrated in FIG. 4 that corresponds to the received sound volume whose sending/received sound ratio has been obtained. The age estimate section 132 estimates an age from the information indicating the identified relationship and the calculated sending/received sound ratio. The age estimate section 132 outputs the estimated age to the minimum audible range estimate section 133.

Based on the age obtained from the age estimate section 132, the minimum audible range estimate section 133 estimates a minimum audible range. The minimum audible range estimate section 133 holds a minimum audible range for each generation, based on the relationship illustrated in FIG. 1.

FIG. 5 is a schematic view illustrating an example of a relationship between age and a minimum audible range. AI illustrated in FIG. 5 represents a minimum audible range of 60's, A2 represents a minimum audible range of 40's, and A3 represents a minimum audible range of 20's. Here, although the relationship between generation and a minimum audible range are provided with intervals of 20 years in the example illustrated in FIG. 5, it may be provided with intervals of 10 years.

As illustrated in FIG. 5, when the generation goes up higher, the minimum audible range becomes higher, which means it is harder to hear. The minimum audible range estimate section 133 holds data that indicates the relationship between generation and a minimum audible range as illustrated in FIG. 5 to obtain a minimum audible range corresponding to the age obtained from the estimate section 132. The minimum audible range estimate section 133 outputs the obtained minimum audible range to the hearing compensation section 105.

Other than the relationship between generation and a minimum audible range, a hearing reduction quantity for each generation may be used. Also, a minimum audible range or a hearing reduction quantity based on gender may be used. For a difference of hearing characteristics by gender, see p. 72-73 of "Building Environment for Aged People", edited by Architectural Institute of Japan, 1994/01/10, Shokokusha Publishing Co., Ltd.

Returning to FIG. 2, the noise estimate section 104 estimates noise of the surroundings from the sending sound. For example, the noise estimate section 104 estimates noise from the average power S_ave of the sending sound of a current frame.

The noise estimate section 104 compares the average power S_ave of the sending sound and a threshold value TH. If S_ave $\geq$ TH, the noise estimate section 104 does not update the noise quantity. If S_ave < TH, the noise estimate section 104 updates the noise quantity by the following formula.

$$\texttt{noise\_level(f)} \quad = \quad \alpha \quad \texttt{x} \quad \texttt{S(f)} \quad + \quad \texttt{(1-}\alpha\texttt{)} \quad \texttt{x}$$
$$\texttt{noise\_level(f)} \quad \cdots \quad \texttt{FORMULA (6)}$$

noise_level(f): noise quantity
$\alpha$: constant

Here, an initial value of noise_level(f) is arbitrary. For example, the initial value may be set to 0. Also, $\alpha$ is a constant between 0 and 1. $\alpha$ is set to, for example, 0.1.

The threshold value TH may be set from 40 to 50 dB. The threshold value TH is set smaller than a volume of a human voice because the volume of voices in people's conversation is 70 to 80 dB. The noise estimate section 104 outputs the estimated noise quantity to the hearing compensation section 105.

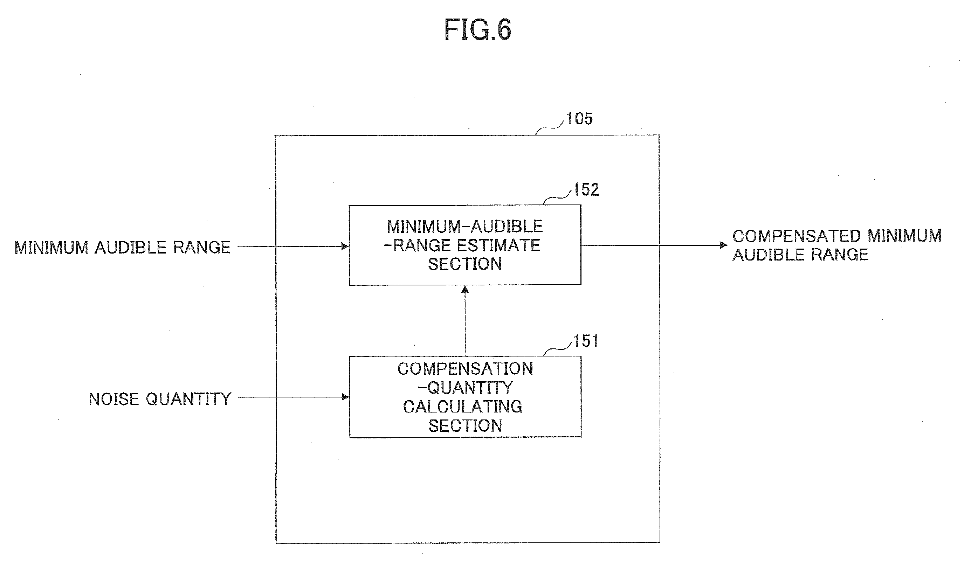

The hearing compensation section 105 compensates hearing (for example, a minimum audible range) based on the minimum audible range obtained from the hearing estimate section 103 and the noise quantity obtained from the noise estimate section 104. FIG. 6 is a block diagram illustrating an example of a configuration of the hearing compensation section 105. The hearing compensation section 105 includes a compensation-quantity calculating section 151 and a minimum audible range compensating section 152.

The compensation-quantity calculating section 151 calculates a compensation quantity in response to the noise quantity obtained from the noise estimate section 104. The compensation-quantity calculating section 151 outputs the noise quantity to the minimum audible range compensating section 152.

The minimum audible range compensating section 152 compensates the minimum audible range based on the minimum audible range obtained from the hearing estimate section 103 and the compensation quantity obtained from the compensation-quantity calculating section 151. The minimum audible range compensating section 152, for example, adds the obtained compensation quantity to the obtained minimum audible range.

In the following, a concrete example of compensation of the minimum audible range will be described.

(Example 1)

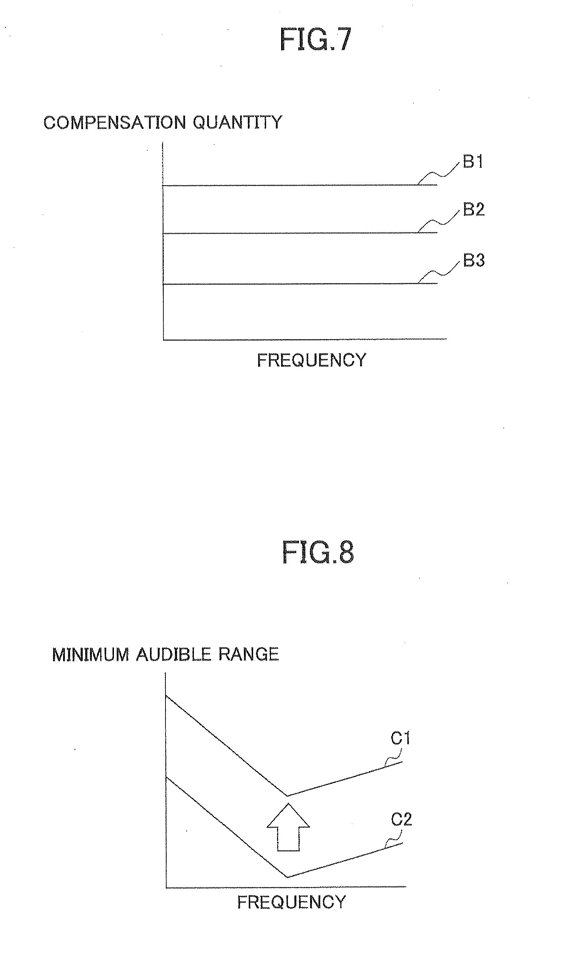

The compensation-quantity calculating section 151 holds a compensation quantity suited to a noise quantity. FIG. 7 is a schematic view illustrating an example of a relationship between noise quantity and compensation quantity. In an example illustrated in FIG. 7, noise is classified into three levels, loud, normal, and soft, and corresponding compensation quantities to the levels are illustrated. In an example illustrated in FIG. 7, B1 represents a compensation quantity of a spectrum whose noise quantity is loud, B2 represents a compensation quantity of a spectrum whose noise quantity is normal, and B3 represents a compensation quantity of the spectrum whose noise quantity is soft.

The compensation-quantity calculating section 151 determines which noise level corresponds to the obtained noise quantity with a determination using a threshold value or the like, to obtain a compensation quantity in response to the determination result from the relationship illustrated in FIG. 7. The compensation-quantity calculating section 151 outputs the obtained compensation quantity to the minimum audible range compensating section 152.

The minimum audible range compensating section 152 adds the compensation quantity obtained from the compensation-quantity calculating section 151 to the minimum audible range obtained from the hearing estimate section 103. FIG. 8 is a schematic view illustrating an example of minimum audible ranges before and after compensation. In an example illustrated in FIG. 8, C1 represents a minimum audible range after compensation and C2 represents a minimum audible range before compensation.

The minimum audible range compensating section 152 obtains a minimum audible range after compensation (C1 illustrated in FIG. 8) by adding to either of the compensation quantity B1 to B3 in FIG. 7 to the minimum audible range of C2 illustrated in FIG. 8. The minimum audible range compensating section 152 outputs the compensated minimum audible range H'(f) to the spectrum compensation-quantity calculating section 106. The compensated minimum audible range H'(f) is, for example, C1 illustrated in FIG. 8.

(Example 2)

The compensation-quantity calculating section 151 calculates a compensation quantity by multiplying the noise quantity, or noise_level(f) which has been obtained from the noise estimate section 104, by constant β. β is a constant set to, for example, 0.1. The compensation-quantity calculating section 151 outputs the calculated compensation quantity to the minimum audible range compensating section 152.

The minimum audible range compensating section 152 obtains a compensated minimum audible range by the following formula.

$$H'(f) = H(f) + β \times noise\_level(f) \quad \cdots FORMULA(7)$$

H'(f): minimum audible range after compensation
H(f): minimum audible range before compensation
β: constant
noise_level(f): noise quantity

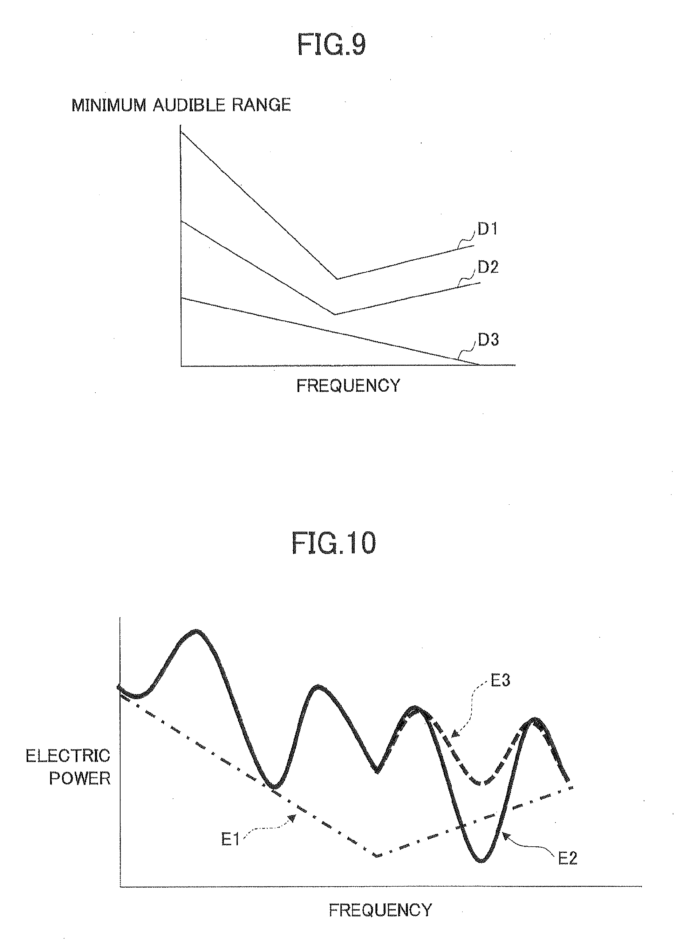

FIG. 9 is a schematic view illustrating an example of a relationship between noise quantity and minimum audible ranges before and after compensation. In an example illustrated in FIG. 9, D1 represents a minimum audible range after compensation, D2 represents a minimum audible range before compensation, and D3 represents a noise quantity.

The minimum audible range compensating section 152 obtains the compensated minimum audible range (D1 illustrated in FIG. 9) by adding a compensation quantity multiplied by the constant β, to the minimum audible range of D2 illustrated in FIG. 9. The minimum audible range compensating section 152 outputs the compensated minimum audible range H'(f) to the spectrum compensation-quantity calculating section 106.

Thus, based on the estimated noise, it is possible to compensate a minimum audible range estimated from an age of a user.

Returning to FIG. 2, the spectrum compensation-quantity calculating section 106 compares the spectrum R(f) of the received sound and the compensated minimum audible range H'(f) to obtain the spectrum compensation quantity G(f). For example, the spectrum compensation-quantity calculating section 106 obtains a spectrum compensation quantity based on the following condition.

$$G(f) = H'(f) - R(f) \ if \ R(f) < H'(f),$$

$$G(f) = 0 \qquad if \ R(f) \geq H'(f)$$

The spectrum compensation-quantity calculating section 106 outputs the obtained spectrum compensation quantity G(f) to the spectrum compensation section 107.

The spectrum compensation section 107 obtains compensated received sound spectrum R'(f) from, for example, the spectrum R(f) of the received sound and the spectrum compensation quantity G(f) by the following formula.

$$R'(f) = R(f) + G(f) \qquad \cdots FORMULA \ (8)$$

FIG. 10 is a schematic view illustrating spectrum compensation. E1 illustrated in FIG. 10 represents a compensated minimum audible range H'(f), E2 represents a received sound spectrum R(f) before compensation, and E3 represents a compensated received sound spectrum R'(f). The spectrum compensation section 107 compensates the received sound spectrum R(f) so that the compensated received sound spectrum R'(f) becomes greater than the minimum audible range H'(f). The spectrum compensation section 107 outputs the compensated spectrum R'(f) of the received sound to the inverse frequency transform section 108.

The spectrum compensation-quantity calculating section 106 may compensate the received sound spectrum only within a predetermined frequency band. The predetermined frequency band is, for example, a low frequency band and/or a high frequency band where hearing tends to be reduced. This is because bands where hearing tends to be reduced are known.

Returning to FIG. 2, the inverse frequency transform section 108 applies an inverse frequency transform (inverse FFT) to the compensated received sound spectrum R'(f) obtained from the spectrum compensation section 107, to obtain compensated received signal r'(t). The compensated received signal r'(t) is output from a speaker to make an output sound.

Thus, the voice control device 1 estimates hearing of a user based on a ratio of volume of sending sound and volume of received sound, and controls the voice in response to the hearing of a user to provide a voice easy to hear for the user automatically.

Also, the voice control device 1 compensates a minimum audible range estimated from the age of the user based on estimated noise to provide a voice even easier to hear for the user.

Here, the noise estimate section 104 and the hearing compensation section 105 are not necessarily required to be configured. In this case, the spectrum compensation-quantity calculating section 106 may calculate the spectrum compensation quantity using the hearing (minimum audible range) estimated by the hearing estimate section 103.

<Operation>

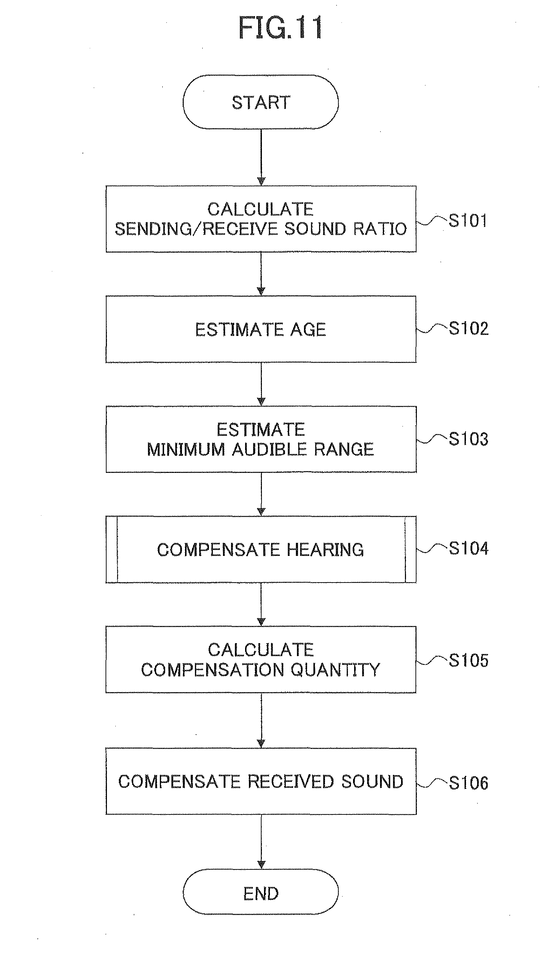

Next, operations of the voice control device 1 will be described according to the embodiment. FIG. 11 is a flowchart illustrating an example of a voice control procedure according to the embodiment.

At Step S101 illustrated in FIG. 11, the sending/received-sound-ratio calculating section 131 calculates a sending/received sound ratio from the volume of a received sound and the volume of a sending sound.

At Step S102, based on the calculated sending/received sound ratio, the age estimate section 132 estimates an age from the information that indicates a relationship between the sending/received sound ratio and age.

At Step S103, based on the estimated age, the minimum audible range estimate section 133 estimates a minimum audible range from the information that indicates a relationship between age (or generation) and minimum audible range.

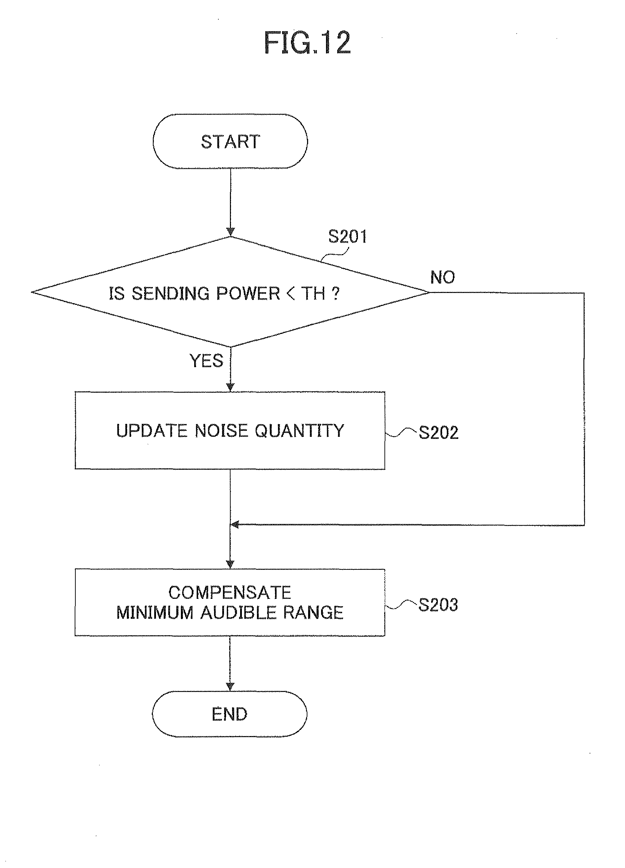

At Step S104, the hearing compensation section 105 compensates the estimated minimum audible range based on noise included in the sending sound. This compensation procedure will be described using FIG. 12.

At Step S105, the spectrum compensation-quantity calculating section 106 calculates the compensation quantity of the received sound spectrum so that the received sound becomes greater than the compensated minimum audible range.

At Step S106, the spectrum compensation section 107 compensates the received signal by adding the calculated compensation quantity, or the like.

Thus, it is possible to provide a voice easy to hear for a user during a call in response to the hearing of the user.

FIG. 12 is a flowchart illustrating an example of a hearing compensation procedure. At Step S201 illustrated in FIG. 12, the noise estimate section 104 determines whether the average electric power of sending sound (also called, "sending power" hereafter) is less than the threshold value TH. If the sending power is less than the threshold value TH (Step S201-YES), Step S202 is taken, or if the sending power is greater than or equal to the threshold value TH (Step S202-NO), Step S203 is taken. If the sending power is less than TH, the noise estimate section 104 determines that the sending sound includes no sound.

At Step S202, the noise estimate section 104 updates the noise quantity using the sending sound spectrum of a current frame by the formula (6).

At Step S203, the hearing compensation section 105 compensates the minimum audible range based on the estimated noise quantity (see FIGs. 8-9).

Thus, it is possible to make a voice easier to hear in response to the surrounding noise by compensating the minimum audible range if the noise quantity of the surroundings is loud. Here, according to the embodiment, compensation of the minimum audible range by a noise quantity is not necessarily required to obtain a sufficient effect.

As above, it is possible to execute voice control in response to a user's hearing without forcing a burden on the user according to the embodiment. Also, it is possible to execute voice control suited to a user because the user is not required to carry out a voice control operation, hence voice control can be done automatically during a call.

Also, the processing by the hearing estimate section 103 may be done at a predetermined timing (once a week, once a month, etc.) so that only hearing compensation by a noise quantity is executed usually. This is because it is not required to execute a hearing estimation every time if the user remains unchanged.

Also, the sending/received-sound-ratio calculating section 131 may calculate a sending/received sound ratio if a sending sound and a received sound include sound (voice). A determination of sound inclusion may be done with a known technology.

For example, in Japanese Patent No. 3849116, voice or non-voice is determined for each frame of an input signal, based on a first voice characterizing quantity calculated using electric power, a zero crossing rate, a peak frequency of a power spectrum, pitch cycle, etc., and a second voice characterizing quantity calculated based on a difference of peak frequency of power spectrum only in high order components. This makes it possible to estimate the hearing of a user based on the volume of a sending sound and the volume of a received sound.

[Modified example]

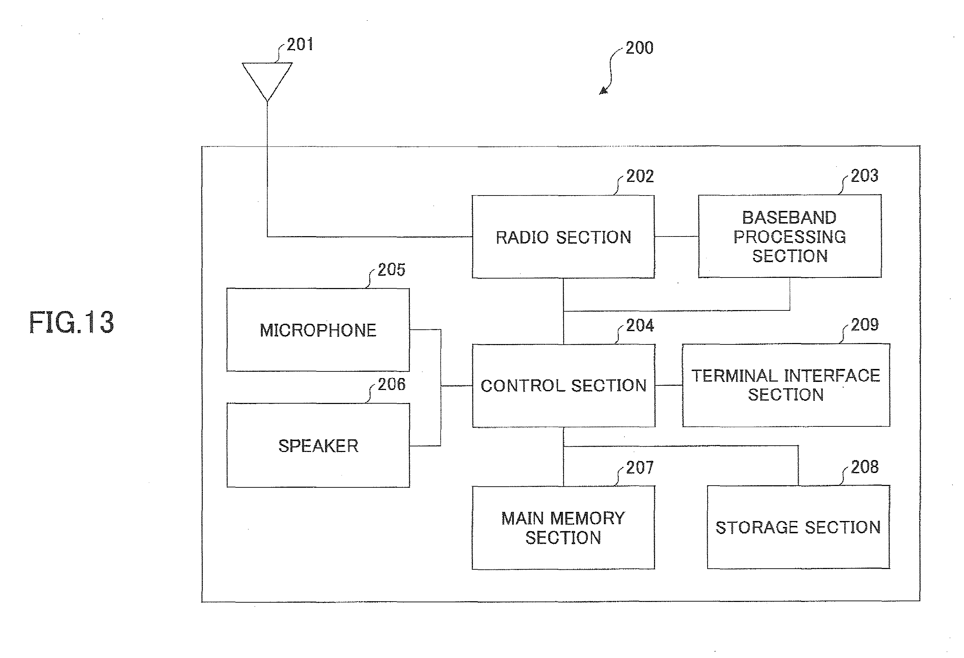

FIG. 13 is a block diagram illustrating an example of hardware of a portable terminal device 200 according to a modified example. The portable terminal device 200 includes an antenna 201, a radio section 202, a baseband processing section 203, a control section 204, a microphone 205, a speaker 206, a main memory section 207, an auxiliary storage section 208, and a terminal interface section 209.

The antenna 201 sends a radio signal amplified by the sending amplifier, and receives a radio single sent from a base station. The radio section 202 applies a D/A conversion to the sending signal spread by the baseband processing section 203, converts it to a high frequency signal by a quadrature modulation, and amplifies the signal by a power amplifier. The radio section 202 amplifies the received radio signal and applies an A/D conversion to the amplified signal to send it to the baseband processing section 203.

The baseband section 203 executes various baseband processing such as an addition of error correcting codes to sending data, data modulation, spread modulation, despreading of a received signal, determination of receiving environment, determination of a threshold value for each channel signal, error correction decoding, etc.

The control section 204 executes radio control such as sending/receiving of a control signal. Also, the control section 204 executes the voice control program stored in the auxiliary storage section 208 to execute voice control according to the embodiment.

The main memory section 207 is a ROM (Read-Only Memory), a RAM (Random Access Memory) or the like, which is a storage device to store or to temporarily store an OS, or the basic software executed by the control section 204, programs such as application software or the like, and data.

The auxiliary storage section 208 is an HDD (Hard Disk Drive) or the like, which is a storage device to store data related to the application software and the like. For example, the information illustrated in FIGs. 4, 5 and 7 is stored in the auxiliary storage section 208.

The terminal interface section 209 executes adapter processing for data, and interface processing with a handset and an external data terminal.

This makes it possible to provide a voice in response to hearing of a user automatically during a call on the portable terminal device 200. Also, it is possible to implement the voice control device 1 according to the embodiments as one or multiple semiconductor integrated circuits in the portable terminal device 200.

Also, the disclosed technology can be implemented not only on the portable terminal device 200, but also on other devices. In the modified example, although the example is explained in which the voice control device is implemented on a portable terminal device, the voice control device described above or the voice control procedures described above are applicable, for example, to a TV telephone conference device and an information processing device having a telephone function, a fixed telephone, and the like.

Also, it is possible to have a computer execute voice control described in the above embodiment by recording a program implementing voice control processing according to the above embodiment in a recording medium.

Also, it is possible to implement the above voice control processing by recording the program on a recording medium and having a computer or a portable terminal device read the recording medium on which the program is recorded. Here, various types of recording media can be used including a recording medium that records information optically, electrically, or magnetically such as a CD-ROM, a flexible disk, an optical magnetic disk and the like, and a semi-conductor memory and the like that records information electrically such as a ROM, a flash memory, and the like.

The embodiments have been described in detail above. Further, the present invention is not limited to these embodiments, but various variations and modifications may be made without departing from the scope of the present invention. Also, it is possible to combine multiple or all of the elements configuring the embodiments described above.

**Claims**

1. A voice control device **characterized by** comprising:

    a hearing estimate section configured to estimate hearing of a user based on a sending/received sound ratio representing a ratio of the volume of sending sound to the volume of received sound;
    a compensation-quantity calculating section configured to calculate a compensation quantity for a received signal of the received sound responsive to the estimated hearing; and
    a compensation section configured to compensate the received signal based on the calculated compensation quantity.

**2.** The voice control device as claimed in claim 1, wherein the hearing estimate section estimates an age of a user from the sending/received sound ratio for estimating a minimum audible range based on the estimated age, wherein the compensation-quantity calculating section obtains the compensation quantity for the received signal so that the received signal is compensated to be greater than the estimated minimum audible range.

**3.** The voice control device as claimed in claim 2, further comprising:

a noise estimate section configured to estimate a noise quantity from the sending sound; and
a hearing compensation section to compensate the minimum audible range based on the estimated noise quantity,
wherein the compensation-quantity calculating section obtains the compensation quantity for the received signal so that the received signal is compensated to be greater than the estimated minimum audible range.

**4.** The voice control device as claimed in any one of claims 1 to 3, wherein the hearing estimate section determines whether the received sound and the sending sound include sound, then obtains the sending/received sound ratio for the received sound and the sending sound if the received sound and the sending sound include the sound.

**5.** The voice control device as claimed in claim 3, wherein the noise estimate section determines whether the received sound includes no sound, then updates the noise quantity if the received sound includes no sound.

**6.** A method for controlling voice in a voice control device, **characterized by** comprising:

estimating hearing of a user based on a sending/received sound ratio representing a ratio of the volume of sending sound to the volume of received sound;
calculating a compensation quantity for a received signal of the received sound to the estimated hearing; and
compensating the received signal based on the calculated compensation quantity.

**7.** A computer-readable recording medium having a program stored therein for causing a computer to execute a method for controlling voice, the method **characterized by** comprising:

estimating hearing of a user based on a sending/received sound ratio representing a ratio of the volume of sending sound to the volume of received sound;
calculating a compensation quantity for a received signal of the received sound responsive to the estimated hearing; and
compensating the received signal based on the calculated compensation quantity.

## FIG.1

EP 2 662 855 A1

# FIG.2

# FIG.3

SENDING
SOUND VOLUME →

RECEIVED
SOUND VOLUME →

131
SENDING/RECEIVED
-SOUND-RATIO
CALCULATING SECTION

132
AGE ESTIMATE
SECTION

133
MINIMUM AUDIBLE
RANGE ESTIMATE
SECTION

→ MINIMUM
AUDIBLE RANGE

103

EP 2 662 855 A1

# FIG.4

ESTIMATED AGE

Y YEARS OLD

X

SENDING/RECEIVED SOUND RATIO

# FIG.5

MINIMUM AUDIBLE RANGE

A1

A2

A3

FREQUENCY

# FIG.6

# FIG.7

COMPENSATION QUANTITY

B1

B2

B3

FREQUENCY

# FIG.8

MINIMUM AUDIBLE RANGE

C1

C2

FREQUENCY

# FIG.9

MINIMUM AUDIBLE RANGE

D1

D2

D3

FREQUENCY

# FIG.10

ELECTRIC
POWER

E3

E1

E2

FREQUENCY

# FIG.11

```
        ( START )
            │
            ▼
┌──────────────────────────┐
│        CALCULATE         │ ～S101
│  SENDING/RECEIVE SOUND RATIO │
└──────────────────────────┘
            │
            ▼
┌──────────────────────────┐
│       ESTIMATE AGE       │ ～S102
└──────────────────────────┘
            │
            ▼
┌──────────────────────────┐
│         ESTIMATE         │ ～S103
│   MINIMUM AUDIBLE RANGE  │
└──────────────────────────┘
            │
            ▼
┌──────────────────────────┐
│    COMPENSATE HEARING    │ ～S104
└──────────────────────────┘
            │
            ▼
┌──────────────────────────┐
│        CALCULATE         │ ～S105
│   COMPENSATION QUANTITY  │
└──────────────────────────┘
            │
            ▼
┌──────────────────────────┐
│  COMPENSATE RECEIVED SOUND │ ～S106
└──────────────────────────┘
            │
            ▼
        (  END  )
```

# FIG.12

```
            ┌─────────────┐
            │    START    │
            └──────┬──────┘
                   │
                   ▼
                  ╱ ╲           S201
                ╱     ╲
              ╱         ╲              NO
            ╱ IS SENDING  ╲ ─────────────────┐
            ╲ POWER < TH ? ╱                  │
              ╲         ╱                     │
                ╲     ╱                       │
                  ╲ ╱                         │
              YES  │                          │
                   ▼                          │
          ┌─────────────────┐                 │
          │ UPDATE NOISE     │   S202          │
          │ QUANTITY         │                 │
          └────────┬─────────┘                 │
                   │                           │
                   │◄──────────────────────────┘
                   ▼
          ┌─────────────────┐
          │   COMPENSATE     │   S203
          │ MINIMUM AUDIBLE  │
          │     RANGE        │
          └────────┬─────────┘
                   │
                   ▼
            ┌─────────────┐
            │     END     │
            └─────────────┘
```

FIG.13

## INTERNATIONAL SEARCH REPORT

| | International application No. |
|---|---|
| | PCT/JP2011/050017 |

**A. CLASSIFICATION OF SUBJECT MATTER**
*G10L21/02*(2006.01)i

According to International Patent Classification (IPC) or to both national classification and IPC

**B. FIELDS SEARCHED**

Minimum documentation searched (classification system followed by classification symbols)
G10L21/02

Documentation searched other than minimum documentation to the extent that such documents are included in the fields searched
Jitsuyo Shinan Koho      1922-1996   Jitsuyo Shinan Toroku Koho    1996-2011
Kokai Jitsuyo Shinan Koho  1971-2011   Toroku Jitsuyo Shinan Koho    1994-2011

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 6-217398 A   (Hitachi, Ltd.),<br>05 August 1994 (05.08.1994),<br>entire text; all drawings<br>(Family: none) | 1-7 |
| A | JP 8-163121 A   (NEC Corp.),<br>21 June 1996 (21.06.1996),<br>entire text; all drawings<br>& US 5777664 A          & EP 713336 A2<br>& DE 69525511 D | 1-7 |
| A | JP 2000-209698 A  (NEC Saitama, Ltd.),<br>28 July 2000 (28.07.2000),<br>entire text; all drawings<br>(Family: none) | 1-7 |

☒ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>    28 January, 2011 (28.01.11) | Date of mailing of the international search report<br>    08 February, 2011 (08.02.11) |
|---|---|
| Name and mailing address of the ISA/<br>    Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2011/050017

C (Continuation).  DOCUMENTS CONSIDERED TO BE RELEVANT

| Category* | Citation of document, with indication, where appropriate, of the relevant passages | Relevant to claim No. |
|---|---|---|
| A | JP 2004-235708 A  (Mitsubishi Electric Corp.), 19 August 2004 (19.08.2004), entire text; all drawings (Family: none) | 1-7 |
| A | JP 8-223256 A  (NEC Corp.), 30 August 1996 (30.08.1996), entire text; all drawings (Family: none) | 1-7 |

Form PCT/ISA/210 (continuation of second sheet) (July 2009)

**REFERENCES CITED IN THE DESCRIPTION**

**Patent documents cited in the description**

- JP 7066767 A
- JP 2004165865 A
- JP 2010081523 A
- JP 3849116 B

**Non-patent literature cited in the description**

- **W. VAN. SUMMERS.** Effects of noise on speech production: acoustic and perceptual analyses. *J. Acoust. Soc. Am.,* September 1988, vol. 84 (3
- Building Environment for Aged People. Shokokusha Publishing Co., Ltd, 10 January 1994, 72-73